# EUROPEAN PATENT APPLICATION

(11) **EP 4 570 255 A1**
(43) Date of publication of application: **18.06.2025**
(21) Application number: 23851128.1
(22) Date of filing: 08.08.2023
(51) Int. Cl.: A61K 31/724, A61L 101/50, C11D 3/48, A61K 31/65, A61K 31/19, A61P 31/02

(54) **SPORICIDAL ANTIMICROBIAL COMPOSITIONS AND USES THEREOF**

(30) Priority: 08.08.2022 BR 102022015619
(71) Applicant: Silva, Renata Moisés Iwamizu, 13254-432 Itatiba (BR)
(72) Inventor: Silva, Renata Moisés Iwamizu, 13254-432 Itatiba (BR)
(74) Representative: Patentree
(86) International application number: PCT/BR2023/050253
(87) International publication number: WO 2024/031162

(57) **Abstract**

The present invention consists of compositions containing cyclic oligosaccharides that function as an encapsulating and germinating agent for spore-forming microorganisms and can also act as an agent for interacting with components of the spore-forming structures of the microorganism. The composition assumes a supramolecular structure favorable to "host-guest" interaction, in association with different antiseptic active ingredients in an inclusion compound, and can be used in formulations containing other excipients, for different uses. More specifically, the present invention relates to compositions with sporicidal and sterilizing antimicrobial activity, with cyclodextrins as the main agents in association with antiseptic active ingredients, in combined methods of action that are highly effective against spore-forming microorganisms, using low concentrations of encapsulated antiseptic active ingredients and having low toxicity.

## Description

The present invention involves compositions containing cyclic oligosaccharides functioning as germinating encapsulating agents for spore-forming microorganisms, while also interacting with components of the spore-forming structures of microorganisms. The composition assumes a supramolecular structure conducive to host-guest interactions, associated with various antiseptic actives in inclusion compounds, potentially integrated with other excipients for diverse applications. More specifically, this invention pertains to sporicidal and sterilizing antimicrobial compositions, where the main agents are cyclodextrins combined with antiseptic actives in synergistic methods, providing high efficacy against spore-forming microorganisms, while utilizing low concentrations of encapsulated antiseptic actives and offering low toxicity.

Microbial resistance has become one of the most significant global public health concerns, especially due to the improper use of antibiotics. Consequently, there has been a significant rise in infections caused by antibiotic-resistant microorganisms with no effective treatment alternatives. Considering the microbial susceptibility hierarchy, spore-forming microorganisms are among the most challenging to eliminate, especially under hostile environmental conditions.

Currently, the specific identification of bacteria responsible for fatal infections is complex and costly, making it difficult to obtain accurate data on the socio-economic impact of infections caused by resistant microorganisms, such as *Clostridium difficile* (*C. difficile*) spores, particularly in underdeveloped countries.

Most cases of infections caused by resistant and spore-forming microorganisms are detected in hospital settings. However, these microorganisms can be present everywhere. Traditional disinfection alternatives effective against spore-forming microorganisms are highly toxic and/or corrosive, in addition to being expensive, significantly impacting hospital and healthcare system budgets. As a result, most hospitals and clinics opt for reduced-spectrum antiseptics for daily disinfection and only resort to broader-spectrum agents in identified contamination zones. This approach directly increases the risk of cross-contamination, creating a favorable environment for the emergence of progressively resistant microorganisms and resulting in more severe diseases.

In the literature, studies on spore-forming microorganisms are primarily focused on bacteria from the *Clostridium* and *Bacillus* genera. Since C. *difficile* is intrinsically resistant to many antibiotics and frequently causes recurrent infections in 20% of cases, this pathogen poses a particular threat to healthcare systems worldwide.

*C. difficile* spores can be transported through the air and on surfaces like fabrics, equipment, hospital waste, hands, and bodies. Transmission typically occurs via the fecal-oral route. Symptoms can be severe, especially for the elderly. It is estimated that 13% of patients remain hospitalized for over two weeks, with 50% of this group staying for more than four weeks. Spores persisting in the environment germinate in the colon, where, in their vegetative state, they release toxins responsible for the clinical aspects of C. difficile-associated disease (CDAD).

Bacterial spores (endospores) are structures produced by bacteria under unfavorable environmental conditions. Endospores exhibit high resistance due to multiple structural layers, each contributing to their resistance against adverse physical and chemical conditions such as extreme heat, pH variations, UV radiation, and antimicrobial chemicals. An endospore can remain dormant for years and convert back into a vegetative cell when conditions become favorable.

The spores of C. *difficile* have, in their structure, from the innermost to the outermost layers: the core, with a low water concentration compared to its vegetative state, where dehydration is associated with resistance to toxic chemicals. Still within the core, high levels of dipicolinic acid (DPA), especially chelated with calcium (Ca-DPA), and the concentration of small acid-soluble proteins (SASPs) protect the microorganism's DNA even after the action of lipases and proteases on the spore's protective layers. These proteins bind to DNA, allowing it to resist ultraviolet radiation, desiccation, and high temperatures. The core is surrounded by an inner membrane rich in peptidoglycans, composed of specific proteins, which are relevant for the release of DPA from the spore core during germination. Surrounding the inner membrane is the cortex, the germ cell wall, a structure of modified peptidoglycans forming a protective mesh that will only be degraded in the presence of specific enzymes. The outer membrane, which surrounds the cortex, is composed of glycosylated proteins similar to collagen, anchored to the basal layer. As an external resistant structure, there is the coat, a proteinaceous layer rich in cysteine and tyrosine that self-assembles into a hexagonal mesh. It is glycosylated and, in some cases, surrounded by an exosporium, the outermost layer of its structure, which is similar to the coat and may be related to the faster germination of C. *difficile.*

The autogermination of the spore is the process of eliminating rigid structures and returning to the vegetative state, that is, the reestablishment of its activities. The process can occur within 5 minutes and takes place when nutritional and environmental conditions become favorable for its development, particularly in the presence of specific germinative agents. In the state of the art, it is reported that sporulated *Bacillus subtilis* and *Clostridium perfringens* are sensitive to nucleosides, sugars, amino acids, and ions to trigger the autogermination process, and that sporulated *C. difficile* germinates in response to a combination of specific bile salts, such as taurocholate, glycocholate, deoxycholate, and L-glycine.

After the germinative agent, whether it is an amino acid, purine derivatives (taurocholate, glycocholate, cholate, and deoxycholate), sugar, or bile salts, binds to the receptors, a series of chain phenomena occur, including the release of monovalent cations (K+, H+, and Na+) and the concentrated dipicolinic acid chelated to the Ca2+ cation (Ca-DPA). The release of Ca-DPA, being an irreversible process, possibly occurs through the closed channels located in the spore's inner membrane, which is partially composed of SpoVA, SpoVAC, and SpoVAD proteins.

Compounds such as quaternary ammonium, organic acids, or alcohols exhibit effective antimicrobial activity but do not demonstrate significant activity as sporicides. Specifically, ethyl alcohol, isopropyl alcohol, and n-propanol are known as inhibitors of sporulation and spore germination, but this action is temporary.

Recent studies show the development of preparation techniques for the application of essential oils as a replacement for environmentally toxic products against fungal spores. However, their application to bacterial spores has not yet been effective.

Cyclodextrins (CDs) are widely used due to their high commercial availability, relatively low cost, low toxicity, known use in the formulation of inclusion compounds, and their role as excipients in drug delivery vehicles and anti-aggregating agents. New formulations with cyclodextrins continue to be reported, generating new interest in science, particularly in the fields of medicine and biomedicine. For many years, cyclodextrins were limited to excipients. However, recent research has shown that these oligosaccharides can no longer be considered merely excipients in formulations. By serendipity, it was discovered that the use of hydroxypropyl-β-cyclodextrin helps reduce the progression of Niemann-Pick disease type C. Based on studies that presented similar results, cyclodextrins were recently classified as active ingredients by the Food and Drug Administration (FDA) and European Medicines Agency (EMA).

Cyclodextrins have characteristics that enable them to act, in the patent in question, in up to three combined ways in the present invention, moving beyond a coadjuvant role and assuming the role of protagonist (active agent), as follows: we will refer to their well-known function of forming inclusion compounds with a wide range of organic and inorganic compounds with antimicrobial action as the encapsulating agent. Particularly in this invention, this capability will allow the antimicrobial active ingredient to be temporarily masked due to the weak bonds formed, concealing the presence of the antiseptic active ingredient in solution. The second role of cyclodextrin in the present invention is due to the fact that its structure is composed of D-glucose units, which, as a sugar, grants it its role as a germinative agent, creating a favorable environment for the microorganism and triggering, in the spore, its autogermination, leading to the self-elimination of the structures that protect it from the external environment. Lastly, cyclodextrin can also function as an interaction agent when it interacts and helps destabilize the spore's structure, due to the hydrophobic characteristic of the internal region of cyclodextrins, which presents a potential interaction region with the lipoproteic base structure of the bacterial wall, such as, for example, calcium-chelated dipicolinic acid (Ca-DPA) and the lipoproteins present in the cortex and exosporium.

The compounds formed as a result of the interaction between the cyclodextrin cavity and different molecules were designated as "host-guest" interaction and later as "inclusion compounds." The host retains non-covalent forces in its cavity, which are weak and allow the entire system to be reversible. The formation of inclusion compounds is the result of the balanced association/dissociation between the free guest and the free host in the compound. This is governed by the formation constant (K_{f}). By solubilizing these compounds, an equilibrium is established between the dissociated and associated structures, which is expressed by the stability constant (Kₐ). The association of the CD and guest molecules, and the dissociation of the formed CD/guest complex, is governed by a thermodynamic equilibrium. The preferred position of the guest in the cavity depends on the steric interactions and functional groups of the guest's structure and the medium in which the inclusion compound is found.

Patent WO2011070456**,** from 2009, titled "Sporocidal composition for *Clostridium difficile* spores," describes a formulation containing a sporicidal composition, which includes about 0.1-20% weight/weight of a germinative agent, about 0.01-75% w/w of an antimicrobial agent, in terms of total dry or wet weight, and is mixed with water to generate a solution with a pH between 3.5 and 9.5. The prior art reports that antimicrobial agents, when combined in a formulation with sodium lauryl sulfate, exhibited a loss of activity; whereas in the presence of germinative agents, such as sodium taurocholate, sodium glycolate, sodium cholate, or glycine, an activity reach of 90% was confirmed. Thus, it highlights the importance of using a germinative agent but does not mention the encapsulating agent with the use of an inclusion compound, such as cyclodextrins.

Patent US2016174566**,** from 2014, titled "Methods, formulation, and kits for bacterial degradation," refers to methodologies, formulations, and kits suitable for decontaminating environments containing bacterial spores through spore degradation. The formulations necessarily contain papain, at least one germinative agent, and optionally one or more additional enzymes. The methods for eliminating bacterial spores include contacting a bacterial spore with the formulation for a duration sufficient to kill the spore or make it susceptible to a reagent. Thus, the document highlights the importance of a germinative agent but conditions the effective action against spores on the presence of papain and does not use cyclodextrins as a germinative encapsulating agent.

In document US20130142856**,** from 2011, titled "Compositions comprising a germinant and antimicrobial agent," it is reported that the germination process to exterminate the microorganism had limited success in the clinical setting. The work presents antibacterial solutions with the presence of germinative agents, such as taurocholate salt and at least two amino acids, to stimulate spore germination and the use of an antibacterial agent, particularly benzalkonium chloride and benzyl alcohol, as they do not inhibit the microorganism's germination. No solution is presented that uses cyclodextrins or any other compound that acts as an encapsulating agent for the active ingredient and could prevent the alteration of the environment favorable to germination from occurring.

Another example is the work presented by Nerandzic & Donskey in 2010, where experiments were conducted on the induction of germination as a strategy to facilitate the elimination of UV-C induced *C. difficile spores* on surfaces, reducing the time and radiation dose required for disinfection of hospital rooms. Furthermore, the potential for initiating germination is reported to improve the ease of eliminating C. *difficile* spores through heat, alcohol, and exposure to environmental oxygen (Nerandzic M.M., Donskey C.J., Triggering Germination Represents a Novel Strategy to Enhance Killing of *Clostridium difficile* Spores, PLoS ONE, 5(8):e12285.).

Although there are already studies presenting the use of encapsulating agents or germinative agents combined with antiseptics against spore-forming bacteria, the use of cyclic oligosaccharides performing this dual function, as a germinative encapsulating agent, is not known. The specific characteristic of cyclodextrins, in particular, allows the protection of the antiseptic active ingredient while simultaneously performing the function of a germinative agent. The purpose of using this class of compounds as a germinative encapsulating agent is to enhance the favorable environment for spore autogermination by protecting the antiseptic active ingredient so that it does not interfere with the favorable environment for germination in the solution. Additionally, when the internal cavity of the cyclodextrin interacts with the structural wall of the microorganism, it accelerates the release of the active ingredient, which will act against the microorganism that is already exposed to the environment and susceptible to the antiseptic action.

Most of the solutions used in the cleaning process in high-risk environments for biological contamination are not effective against C. *difficile* spores. Therefore, the present invention presents a new product that is low in toxicity, non-corrosive, economically viable, and highly effective in preventing the spread of spore-forming pathogenic microorganisms in the environment.

Among the most commonly used biocides in antiseptic products, disinfectants, and preservatives are chlorhexidine and its salts. Their use is due to their effectiveness against a broad spectrum of microorganisms. However, there are no reports indicating satisfactory sporicidal activity of these compounds. Similarly to doxycycline, an antibiotic from the tetracycline class, even though it shows activity against various microorganisms, no satisfactory sporicidal activity has been reported.

The present invention lists natural or synthetic active ingredients with known antimicrobial activity but no efficacy against spore-forming microorganisms, which are incorporated into an inclusion compound, functioning as a germinative encapsulating agent. Cyclodextrin, particularly beta-cyclodextrin, and its structural variations, are used as the encapsulating agent. The results presented show that after the formation of the inclusion compound, the active ingredient, which previously had no sporicidal efficacy, gains activity in eliminating spores.

The properties of cyclodextrins are fully utilized in the present invention. Their ability to establish specific molecular encapsulation interactions through the formation of non-covalent bonds, such as hydrophobic interactions, van der Waals forces and hydrogen bonds, is fundamental to the success of this invention. Since these are weak bonds in the inclusion compounds, the encapsulation strategy initially and the release of the guest only at a later stage create a sequence of activities that are crucial for the effectiveness of the present invention. The active ingredient remains concealed until the moment when spore autogermination occurs, and then the active ingredient is released and fulfills its role, when the microorganism is already vulnerable.

Considering the favorable environment that cyclodextrin can create, which positively contributes to the autogermination of spores, we understand that the higher the formation constant between the antimicrobial active ingredient and cyclodextrin, the more favorable the environment will be, with a lower risk of exposure of the antimicrobial active ingredients in the medium at the initial stage.

Due to the toroidal structure of cyclodextrins, with their hydrophobic cavity and more hydrophilic external surface, an energetically favorable interaction occurs between the compound to be encapsulated and the cyclodextrin cavity. For the present invention, it is possible to use active ingredients where the interaction between the active ingredient and the cyclodextrin cavity exists, but is weaker than the one that will naturally occur between the lipoprotein wall of the microorganism and the cavity.

In the present invention, the inclusion compound combining cyclodextrins with different antiseptic active ingredients, which are known to be ineffective against spores in their free form, demonstrated sporicidal activity, even with significantly low amounts of active ingredient. Thus, cyclodextrin does not play the role of a simple excipient but rather functions as a germinative agent, acting as a Trojan horse mechanism, where the spore does not detect the presence of the antiseptic active ingredient, as the functional groups of the active ingredient are not exposed, maintaining an environment favorable to its germination.

The combination of factors associated with physicochemical parameters such as the formation constant, stability constant, enthalpy, steric effect, and polarity are crucial for the formulation of products effective against spores. Thus, in the present invention, cyclodextrins initially play a fundamental role when they simply protect the antiseptic active ingredient inside their cavity and, later, as a germinative agent, when they provide the microorganism with a false sense of security for germination, and can also act as an interaction agent with the spore wall.

The present invention preferentially used beta-cyclodextrin due to its more rigid structure, with its complete secondary belt, as a result of the greater number of hydrogen bonds formed between the C-2-OH group of one glucopyranoside unit and the C-3-OH group of the adjacent glucopyranose unit (4C1 conformation), which justifies its significantly lower solubility and relatively more hydrophobic cavity compared to the alpha and gamma alternatives.

Thus, the present invention presents the use of cyclodextrin and its variations as germinative agents for spore-forming microorganisms, in addition to acting as carriers or encapsulants for various antimicrobial agents. Furthermore, it presents products with very low toxicity that replace existing alternatives, while also enabling the use of sporicidal products more broadly, directly impacting the increased safety of hospitals against nosocomial infections.

### DETAILED DESCRIPTION OF THE TECHNOLOGY

In particular, the present invention presents the use of cyclodextrins, cyclic oligosaccharides formed by units of D-glucose, more specifically glucopyranoses, as germinative agents for C. *difficile,* making them a potential ingredient in microbiological formulations, especially against its spores.

The technology presents sporicidal antimicrobial compositions that comprise a germinative encapsulating agent, which is a cyclic oligosaccharide, at least one antiseptic active ingredient in inclusion compound with the cyclic oligosaccharide, and excipients.

The cyclic oligosaccharide is cyclodextrin or its structural variations, or a mixture thereof. The structural variations of cyclodextrin can include compounds such as beta-cyclodextrin, hydroxypropyl-beta-cyclodextrin, sodium beta-dexsulfobutyl ether, hydroxy methyl-beta-cyclodextrin, methyl-beta-cyclodextrin, 2,6-dimethyl-beta-cyclodextrin, and hydroxyethyl-beta-cyclodextrin.

The antiseptic active ingredient can be a cationic, anionic, zwitterionic, or neutral antimicrobial active ingredient, as long as such actives can form inclusion complexes with cyclic oligosaccharides and have their functional groups with antimicrobial activity temporarily protected within the formed inclusion complex.

Among the various alternatives already known in the literature, we present as examples the classes of bis-biguanides, such as chlorhexidine or its acetate, gluconate, or digluconate salts, and the classes of tetracyclines, such as doxycycline, eracycline, monocycline, omadacycline, or combinations of these.

Considering the role of cyclodextrin in this patent, which goes beyond the role of an encapsulating agent, additional cyclodextrins may be added without forming an inclusion compound, with the aim of acting as a germinant in the formulation and/or their structural variations or combinations thereof, which may range from 0.01 to 20% w/w.

Some of the halogenated antimicrobial compounds that have been specially studied, with broad-spectrum antimicrobial activity and that are useful in the compositions of the present invention, include 1,1'-hexamethylene-bis-(5-(p-chlorophenyl)biguanide), commonly known as chlorhexidine and its salts, such as with hydrochloric, acetic, and gluconic acids. The digluconate salt is highly soluble in water, about 70% in water, and the diacetate salt has a solubility of about 1.8% in water.

Other useful biguanide compounds include Cosmoci^{®} CQ^{®} and Vantocil^{®} IB, which contain poly(hexamethylene biguanide) hydrochloride. Other useful cationic antimicrobial agents include bis-biguanide alkanes. The water-soluble salts of the aforementioned compounds include chlorides, bromides, sulfates, alkylsulfonates such as methylsulfonate and ethylsulfonate, phenylsulfonates such as p-methylphenylsulfonates, nitrates, acetates, gluconates, and similar compounds.

Examples of suitable bis-biguanide compounds include chlorhexidine; 1,6-bis-(2-ethylhexylbiguanido-hexano) dichloride; tetra-hydroxychloride of 1,6-di-(N1,N1'-phenylbiguanido-N5,N5')-hexano; 1,6-di-(N1,N1'-phenyl-N1,N1'-methylbiguanido-N5,N5')-hexano dichloride; 1,6-di-(N1,N1'-o-chlorophenylbiguanido-N5,N5')-hexano dichloride; 1,6-di-(N1,N1'-2,6-dichlorophenylbiguanido-N5,N5')-hexano dichloride; 1,6-di-[N1,N1'-Beta-(p-methoxyphenyl)biguanido-N5,N5']-hexano dichloride; 1,6-di-(N1,N1'-Alpha-methyl-beta-phenylbiguanido-N5,N5')-hexano dichloride; 1,6-di-(N1,N1'-p-nitrophenylbiguanido-N5,N5')-hexano dichloride; omega'-di-(N1,N1'-phenylbiguanido-N5,N5')-di-dichloride of n-propyl ether; omega'-di-(N1,N1'-p-chlorophenylbiguanido-N5,N5')-tetrahydroxychloride of di-n-propyl ether; tetra-hydroxychloride of 1,6-di-(N1,N1'-2,4-dichlorophenylbiguanido-N5,N5')-hexano; 1,6-di-(N1,N1'-p-methylphenylbiguanido-N5,N5')-hexano dichloride; tetra hydroxychloride of 1,6-di-(N1,N1'-2,4,5-trichlorophenylbiguanido-N5,N5')-hexano; 1,6-di-[N1,N1'-Alpha-(p-chlorophenyl)ethylbiguanido-N5,N5']-hexano dichloride; omega: omega-di-(N1,N1'-p-chlorophenylbiguanido-N5,N5') dichloride of m-xylene; 1,12-di-(N1,N1'-p-chlorophenylbiguanido-N5,N5')-dodecane dichloride; 1,10-di-(N1,N1'-phenylbiguanido-N5,N5')-tetrahydroxychloride of decane; 1,12-di-(N1,N1'-phenylbiguanido-N5,N5') dodecane tetrahydroxychloride; 1,6-di-(N1, N1'-o-chlorophenylbiguanido-N5,N5')-hexano dichloride; 1,6-di-(N1,N1'-p-chlorophenylbiguanido-N5,N5')-hexano tetrahydroxychloride; ethylene bis(1-tolylbiguanide); ethylene bis(p-tolylbiguanide); ethylene bis(3,5-dimethylphenylbiguanide); ethylene bis(p-terc-amilphenylbiguanide); ethylene bis(nonylphenylbiguanide); ethylene bis(phenylbiguanide); ethylene bis(N-butylphenylbiguanide); ethylene bis(2,5-diethoxyphenylbiguanide); ethylene bis(2,4-dimethylphenylbiguanide); ethylene bis(o-diphenylbiguanide); ethylene bis(ami-naphthylbiguanide mixed); N-butyl ethylene bis(phenylbiguanide); bis(o-tolylbiguanide) of trimethylene; N-butyl trimethylene bis(phenylbiguanide), and the pharmaceutically acceptable salts corresponding to all of the above, such as acetates; gluconates; hydrochlorides; bromides; citrates; bissulphites; fluorides; polyaleates; N-alkylalkylsarcosinates; phosphites; hypophosphites; perfluorooctanoates; silicates; sorbates; salicylates; maleates; tartrates; fumarates; ethylenediaminetetraacetates; iminodiacetates; cinnamates; thiocyanates; arginates; pyromellitates; tetracarboxybutyrates; benzoates; glutarates; monofluorophosphates; and perfluoropropionates and their mixtures.

The antimicrobials and their respective inclusion compounds with cyclodextrins in molar ratios of 1:2 to 1:10, or higher molar ratios, preferred from this group, include tetrahydrochloride of 1,6-di-(N1, N1'-phenylguanido-N5,N5')-hexane; Diclorhydrate of 1,6-di-(N1,N1'-o-chlorophenylguanido-N5,N5')-hexane; Diclorhydrate of 1,6-di-(N1,N1'-2,6-dichlorophenylguanido-N5,N5')-hexane; Tetrahydrochloride of 1,6-di-(N1,N1'-2,4-dichlorophenylguanido-N5,N5')-hexane; Diclorhydrate of 1,6-di-[N1,N1'-.Alpha.-(p-chlorophenyl)ethylguanido-N5,N5']-hexane; Omega: Diclorhydrate of Mega.'di-(N1,N1'-p-chlorophenylguanido-N5,N5')-m-xylene; Diclorhydrate of 1,12-di-(N1,N1'-p-chlorophenylguanido-N5,N5') dodecane; Diclorhydrate of 1,6-di-(N1,N1'-o-chlorophenylguanido-N5,N5')-hexane; 1,6-di-(N1,N1'-p-chlorophenylguanido-N5,N5')-hexane tetrahydrochloride; and mixtures thereof. More preferably, diclorhydrate of 1,6-di-(N1,N1'-o-chlorophenylguanido-N5,N5')-hexane; Diclorhydrate of 1,6-di-(N1,N1'-2,6-dichlorophenylguanido-N5,N5')-hexane; Tetrahydrochloride of 1,6-di-(N1,N1'-2,4-dichlorophenylguanido-N5,N5')-hexane; Diclorhydrate of 1,6-di-[N1,N1'-Alpha-(p-chlorophenyl)ethylguanido-N5,N5]-hexane; Omega: Mega.'di-(N1,N1'-p-chlorophenylguanido-N5,N5')-m-xylene; Diclorhydrate of 1,12-di-(N1,N1'-p-chlorophenylguanido-N5,N5')-dodecane; Diclorhydrate of 1,6-di-(N1,N1'-o-chlorophenylguanido-N5,N5')-hexane; 1,6-di-(N1,N1'-p-chlorophenylguanido-N5,N5')-hexane tetrahydrochloride; and mixtures of these.

Tetracyclines are compounds belonging to the group of natural or semisynthetic antibiotics that can be used in conjunction with cyclodextrins to obtain inclusion compounds.

A wide variety of quaternary compounds can also be used as antimicrobial and antiviral agents for compositions with cyclodextrins and their inclusion compounds in molar ratios of 1:3 to 1:10 or higher molar ratios of quaternary ammonium compounds:cyclodextrins in the present invention. Non-limiting examples of useful quaternary compounds include: benzalkonium chlorides and/or substituted benzalkonium chlorides, such as Barquat^{®} available on the market, Maquat^{®}, Variquat^{®}, and Hyamine^{®}; di-(C6-C14)-alkyl-dialkyl quaternaries of short chain (C1-4 alkyl and/or hydroxyalkyl), such as Bardac^{®} products; N-(3-chloroallyl)hexamine chlorides, such as Dowicide^{®} and Dowicil^{®}; benzethonium chloride such as Hyamine^{®} 1622 from Rohm & Haas; methylbenzethonium chloride represented by Hyamine^{®} 10×; cetylpyridinium chloride, such as Cepacol^{®} chloride. Preferably, the dialkyl quaternary compounds are di-(C8-C12) dialkyl dimethyl ammonium chloride, such as didecyl dimethyl ammonium chloride (Bardac 22) and dioctyl dimethyl ammonium chloride (Bardac 2050).

Among the active compounds, another alternative are saturated chain organic acids with carbon atom numbers between 2 and 8, such as acetic acid, propanoic acid, butanoic acid, 2-methylpropanoic acid, pentanoic acid, 2-methylbutanoic acid, 3-methylbutanoic acid, dimethylpropanoic acid, hexanoic acid, 2-methylpentanoic acid, 3-methylpentanoic acid, 4-methylpentanoic acid, 2,2-dimethylbutanoic acid, 3,3-dimethylbutanoic acid, 2-ethylbutanoic acid, 2,3-dimethylbutanoic acid, heptanoic acid, 2-methylhexanoic acid, 3-methylhexanoic acid, 4-methylhexanoic acid, 5-methylhexanoic acid, 3,4-dimethylpentanoic acid, 2,4-dimethylpentanoic acid, 2,3-dimethylpentanoic acid, 2,2-dimethylpentanoic acid, 3,3-dimethylpentanoic acid, 4,4-dimethylpentanoic acid, 2,2,3-trimethylbutanoic acid, 2,3,3-trimethylbutanoic acid, 2-ethylpentanoic acid, 2-ethyl-3-methylbutanoic acid, octanoic acid, 2-methylheptanoic acid, 3-methylheptanoic acid, 4-methylheptanoic acid, 5-methylheptanoic acid, 6-methylheptanoic acid, 2-ethylhexanoic acid, 3-ethylhexanoic acid, 4-ethylhexanoic acid, 2,2-dimethylhexanoic acid, 3,3-dimethylhexanoic acid, 4,4-dimethylhexanoic acid, 5,5-dimethylhexanoic acid, 2,3-dimethylhexanoic acid, 2,4-dimethylhexanoic acid, 2,5-dimethylhexanoic acid, 3,4-dimethylhexanoic acid, 3,5-dimethylhexanoic acid, 4,5-dimethylhexanoic acid, 2,3,4-trimethylpentanoic acid, 3,4,4-trimethylpentanoic acid, 2,3,3-trimethylpentanoic acid, 2,4,4-trimethylpentanoic acid, 2,2,4-trimethylpentanoic acid, 2,2,3-trimethylpentanoic acid, 2-propylpentanoic acid, 3-ethylhexanoic acid, 2-isopropylpentanoic acid, 3-ethyl-4-methylpentanoic acid, 3-ethyl-2-methylpentanoic acid, 3-ethyl-3-methylpentanoic acid, 2,2,3,3-tetramethylbutanoic acid, 2-ethyl-2,3-dimethylbutanoic acid, 2,2-diethylbutanoic acid, or mixtures thereof.

The antiseptic active ingredient can also be selected from groups comprising the classes of saturated or unsaturated organic acids, with a main chain of carbon atoms between 2 and 8, or combinations of these acids, at a concentration of 0.01 to 30% w/v, preferably at a concentration of 0.05 to 20% w/v.

The antiseptic active ingredient is also selected from groups comprising the classes of essential oils derived from natural bases such as thyme, eucalyptus, oregano, basil, mint, peppermint, rosemary, wild rosemary, pequi, geranium, citronella, palmarosa, pitanga, cypress, tea tree, ginger, lemongrass, lemon grass, or propolis, or combinations thereof, at concentrations between 0.05 and 20% w/w.

Essential oils extracted by steam distillation, maceration, solvent extraction, enfleurage, supercritical gases, or microwaves, and their distilled derivatives may or may not be added to the final product formulation as active ingredients or excipients.

Excipients may be humectants, emulsifiers, preservatives, thickeners, sweeteners, stabilizers, colorants, antioxidants, surfactants, and/or flavoring agents at concentrations ranging from 0.02% to 70.0% w/w.

The use of the sporicidal composition can be in aqueous solution, powder, paste, wet gel, xerogels, aerosols, spray, or foam, tablets, capsules, incorporated into cellulose materials, polymers, or fabrics. They can be used in the production of disinfectant sanitizers for surface disinfection, for the production of formulations for topical use, or for environmental treatment, surface hygiene, air sanitization, or in air purification equipment. The surface can be selected from the group comprising epithelial tissues, skin, fingers, nails, hair, mammary glands, perineal region, genitals, rectum, or mucous membranes.

Additionally, they have pharmacological applications for the treatment of gastrointestinal diseases or diseases caused by spore-forming microorganisms, acting as antibiotics in the pharmaceutical form of capsules, oral tablets (gastroresistant release), or suppositories for rectal use. For use as a medication, the inclusion complex formed may also be nanoencapsulated or encapsulated in other drug delivery structures to target the active ingredient to the desired site of action.

The solution presented in this invention can also be used in the veterinary field, such as in veterinary or agricultural medicines, for application in animal feed or agricultural pesticides.

The present technology can be better understood through the following examples, which are not limiting.

### Example 1. Processes for preparing the compositions of the inclusion compounds of chlorhexidine digluconate:cyclodextrin in a molar ratio of 1:1 to 1:10.

The process for obtaining the inclusion compound includes the steps following the procedure reported in the literature (Cortez et al., The chlorhexidine:β-Cyclodextrin Inclusion compounds: preparation, characterization, and microbial evaluation. Journal of Inclusion Phenomena and Macrocyclic Chemistry, 40, 2001, 97 - 302).

The solutions with the inclusion compounds were obtained by adding the chlorhexidine salt to an aqueous solution containing an encapsulant from the cyclodextrin family (beta-cyclodextrin, hydroxypropyl-beta-cyclodextrin, betadexsulfobutyl ether sodium, hydroxymethyl-beta-cyclodextrin, methyl-beta-cyclodextrin, 2,6-dimethyl-beta-cyclodextrin, and hydroxyethyl-beta-cyclodextrin) at 60°C, maintaining the molar ratios between 1:1 and 1:10.

After a period of 30 minutes to 2 hours of stirring, the inclusion compound was obtained in powder form using Spray Dryer drying.

### Example 2. The preparation process of the antimicrobial system with inclusion compound and organic acid.

The preparation process of the inclusion compound and organic acid system was carried out following the following formulations:

In an aqueous solution containing the organic acid, such as acetic acid, at concentrations ranging from 0.01% to 20.0%, cyclodextrin powder was added in the appropriate molar ratio for the formulation, to obtain a solution in which the compound is present at a concentration between 0.01% and 20.0%.

After a period of 30 minutes to 2 hours of stirring, the product was obtained in powder form using Spray Dryer or Lyophilizer.

### Example 3. Study of the microbiological activity of the inclusion compound chlorhexidine digluconate:β-cyclodextrin against the spore-forming bacterium Clostridium difficile using the Kill Time Test methodology for 5 and 60 minutes.

Tests were conducted to evaluate the action spectrum of the inclusion compounds in the molar ratios of chlorhexidine digluconate:β-cyclodextrin of 1:2 (L3), 1:3 (A9), by monitoring the microorganism population within 5 and 60 minutes at a fixed temperature of 35 ºC. The direct plating method was performed on TSA for bacteria and HC for yeast (DE QUA 1502- ASTM E2315-16, Standard Guide for Assessment of Antimicrobial Activity Using a Time-Kill Procedure, ASTM International, West Conshohocken, PA, 2016).

The initial microorganism count was 10^6 to 10^8 CFU/g or mL. Table 1 presents the data on activity against the spore-forming bacterium *C. difficile* (ATCC 9689), showing the log data of colony-forming units per gram after exposure (CFU/g or mL) and the reduction factor based on the initial baseline count (% RF).

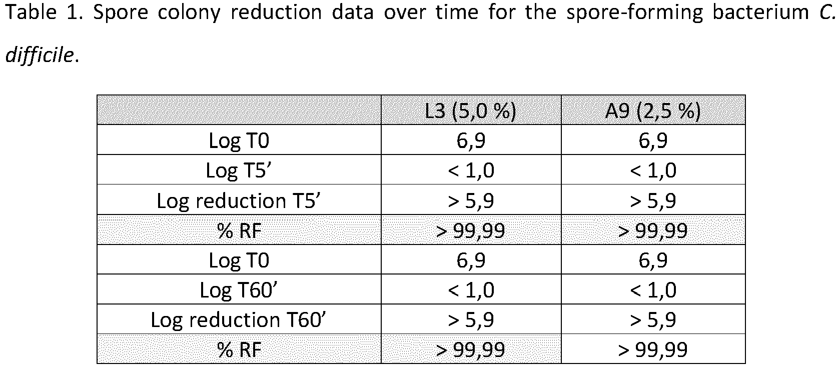

Based on the obtained data, it can be stated that the tested inclusion compounds exhibit antimicrobial activity against spore-forming Clostridium difficile, producing a 99.99% reduction after 5 and 60 minutes of contact with the microorganism.

### Example 4. Study of the microbiological activity of the inclusion compound chlorhexidine digluconate:β-cyclodextrin at a molar ratio of 1:3 (A9) against spore-forming Clostridium spp. using the Kill Time Test methodology for 5, 30, and 60 minutes.

The activity studies of the inclusion compound A9 were conducted using the AOAC - 966.04:2016 methodology. The activities of the compound were determined at 5, 30, and 60-minute intervals, at concentrations of 1%, 2%, and 2.5% (w/v).

The sanitizer used as a control has the following formulation: inclusion compound A9 (2.5%), alkyl dimethyl benzyl ammonium chloride and dodecyl dimethyl ammonium chloride (0.5%), ethoxylated fatty alcohol C9-C11 (2%), methylisothiazolinone and methylchloroisothiazolinone (0.0015%), 2-bromo-nitropropane-1,3-diol (0.0082%), aqua (qsp).

The data on the antimicrobial activity of the materials against spore-forming C. *difficile* bacteria are presented in Table 2.

**Table 2. Disinfection time data for spore-forming C. difficile bacteria after contact with the compounds.**

| Sample code (sample concentration w/v) | Action time | Result (elimination of 10 out of 10 carriers) |
|---|---|---|
| L3 (1,0 %) | 30 min | 10 |
| L3 (2,0 %) | 5 min | 10 |
| A9 (1,0 %) | 15 min | 10 |
| A9 (2,0 %) | 15 min | 10 |
| A9 (2,5 %) | 5 min | 10 |
| A9 (2,5 %) | 30 min | 10 |
| A9 (2,5 %) | 60 min | 10 |
| Sanitizer (control) | 30 min | 4 |

It can be observed that the inclusion compound, whether in the molar ratio of 1:2 (L3) or 1:3 (A9), shows satisfactory activity at different times and concentrations. For A9, at a concentration of 2.5%, activity was already observed starting at 5 minutes. For L3, a concentration of 2% was sufficient to show activity at the shortest time (5 minutes).

It is important to note that the sanitizer, which contains the inclusion compound A9 at a concentration of 2.5%, lost sporicidal activity when formulated with other antimicrobial agents, compared to the same time and concentration of the A9 active in aqueous solution. The result clearly shows that the presence of other antimicrobial agents in the final product formulation leads to a reduction in sporicidal activity against the spore of the tested bacterium (sporulated C. *difficile),* which can be explained by the fact that the favorable environment for spore germination, provided by cyclodextrin, was altered.

### Example 5. Evaluation of the sporicidal activity of the inclusion compound doxycycline:beta-cyclodextrin at a molar ratio of 1:4 against the bacterium Clostridium difficile sporulated.

The activity study against the spore of the bacterium Clostridium difficile was conducted using a 2.5% (w/v) inclusion compound solution over a 30-minute action period against the microorganism, following the AOAC - 966.04:2016 methodology.

The inclusion compound showed satisfactory activity in the evaluation, demonstrating that its activity was confirmed by the elimination of the microorganism in a sufficient quantity from the carriers used in the test (Table 3).

**Table 3. Evaluation of the sporocidal activity of the doxycycline:cyclodextrin compound against C. difficile.**

| Sample code (sample concentration w/v) | Action time | Result (elimination of 10 out of 10 carriers) |
|---|---|---|
| doxycycline:cyclodextrin (2,5 %) | 30 min | 10 |

The result shows that the inclusion compound (doxycycline:cyclodextrin) demonstrated sporicidal activity.

## Claims

1. Antimicrobial sporicidal compositions, **characterized by** comprising a germinating encapsulating agent, which is a cyclic oligosaccharide, at least one antiseptic active ingredient in inclusion compound with the cyclic oligosaccharide, and excipients.

2. Antimicrobial sporicidal compositions, according to claim 1, **characterized by** the germinating encapsulating agent cyclic oligosaccharide being the cyclodextrin or its structural variations, or a mixture thereof.

3. Antimicrobial sporicidal compositions, according to claims 1 and 2, **characterized by** the structural variations of cyclodextrin being comprised by the compounds beta-cyclodextrin, hydroxypropyl-beta-cyclodextrin, beta-dexsulfobutylether sodium, hydroxy-methyl-beta-cyclodextrin, methyl-beta-cyclodextrin, 2,6-dimethyl-beta-cyclodextrin, and hydroxyethyl-beta-cyclodextrin.

4. Antimicrobial sporicidal compositions, according to claim 1, **characterized by** the antiseptic active ingredient being a cationic, anionic, zwitterionic, or neutral antimicrobial active ingredient, such as bis-biguanide classes like chlorhexidine or its acetate, gluconate, or digluconate salts, tetracycline classes like doxycycline, eracycline, monocyline, omadacycline, or a combination thereof.

5. Antimicrobial sporicidal compositions, according to claims 1 to 4, **characterized by** the antiseptic active ingredient and the germinating agent being in molar proportions of 8:1 to 1:10, respectively.

6. Antimicrobial sporicidal compositions, according to claims 1 and 4, **characterized by** comprising 0.01 to 20% w/w in excess of the germinating agent in the product and/or its structural variations or combinations thereof.

7. Antimicrobial sporicidal compositions, according to claims 1 and 4, **characterized by** comprising excipients such as humectants, emulsifiers, preservatives, thickeners, sweeteners, stabilizers, colorants, antioxidants, surfactants, and/or flavoring agents in concentrations between 0.02 and 70.0% w/w.

8. Antimicrobial sporicidal compositions, according to claims 1 and 4, **characterized by** the antiseptic active ingredient being selected from the groups comprising the classes of organic acids with saturated or unsaturated chains, with the main chain having a number of carbon atoms between 2 and 8, or combinations of these acids in a concentration of 0.01 to 30% w/v, preferably in a concentration of 0.05 to 10% w/v.

9. Antimicrobial sporicidal compositions, according to claims 1 and 4, **characterized by** the antiseptic active ingredient being selected from the groups comprising the classes of essential oils like thyme, eucalyptus, oregano, basil, mint, peppermint, rosemary, wild rosemary, pequi, geranium, citronella, palmarosa, pitanga, cypress, melaleuca, ginger, lemongrass, lemon grass, or propolis or a combination thereof in concentrations between 0.05 and 20% w/w.

10. Use of sporicidal compositions as defined in any of claims 1 to 9, **characterized by** being in an aqueous solution, powder, paste, wet gel, xerogels, aerosols, spray, or foam, tablets, capsules, inserted into cellulosic, polymeric materials, or fabrics.

11. Use of sporicidal compositions, according to claim 10, **characterized by** being a sanitizer for surface disinfection, for the production of formulations for topical use, or for environmental treatment, surface sanitation, air sanitation, or air purification equipment.

12. Use of sporicidal compositions, according to claim 10, **characterized by** the surface being selected from the group comprising epithelial tissues, skin, fingers, nails, hair, mammary glands, perineal region, genitals, rectum, or mucous membranes.

13. Use of sporicidal compositions, according to claim 10, **characterized by** being for pharmacological application, as antibiotics, for oral, rectal, genital, ocular, or nasal use.

14. Use of sporicidal compositions, according to claim 10, **characterized by** being active against the spore-forming bacterium *Clostridium difficile.*

15. Use of sporicidal compositions, according to claim 10, **characterized by** being for application in veterinary medicine, or agricultural, in animal feed, or agricultural protector.
